# EUROPEAN PATENT APPLICATION

(11) **EP 1 510 175 A1**
(43) Date of publication of application: **02.03.2005**
(21) Application number: 04104079.1
(22) Date of filing: 30.07.2002
(51) Int. Cl.: A61B 5/22, A61B 5/00, A63B 24/00

(54) **Exercise manager program**

(62) Divisional of application: 02447148.4
(71) Applicant: Kostucki, Willy, 1190 Bruxelles (BE)
(72) Inventor: Kostucki, Willy, 1190 Bruxelles (BE)
(74) Representative: Quintelier, Claude

(57) **Abstract**

A device and a method for adapting parameters of a training device comprising the steps of composing a training cycle, determining a number of subsequent training cycles, determining said target heart rate for each phase and regulation parameters for the training device, forming a training data set, supplying said training data set to said training device, monitoring of a biomedical parameter, collecting a data set captured during said training session, recording a session data set, and comparing the session data set of said performed session with the training data set and verifying an effective performing of the training session.

## Description

The present invention relates to a method for adapting parameters of a training device comprising the steps of:
- composing a training cycle comprising :
   - a body warming-up phase having a warming-up phase time duration and a warming-up phase target heart rate to be determined by a physician,
   - at least one body training phase having a training phase time duration, an exercise profile and a training phase target heart rate to be determined by said physician,
   - a body recovery phase having a recovery phase time duration and a recovery phase target heart rate to be determined by said physician;
- determining a number of subsequent training cycles, said number of cycles forming a training session having a session time duration;
- determining said warming up phase target heart rate, said training phase target heart rate and said recovery phase target heart rate for a user and regulation parameters for the training device;
- forming a training data set by associating said warming-up phase time duration, said warming-up phase target heart rate, said training phase time duration, said exercise profile, said training phase target heart rate, said recovery phase time duration, said recovery phase target heart rate, said number of subsequent training cycle, said session time duration and said regulation parameters;
- supplying said training data set to said training device;
- converting said training data set in order to form a training set-up using said regulation parameters for regulating a work load to be applied to said training device;
- monitoring of a biomedical parameter of said user, in particular a heart rate, when performing said training session;
- collecting a data set captured during said training session, said captured data set comprising:
   - effective duration of each phase, each phase being either said warming up phase, said at least one training phase and said recovery phase,
   - heart rate of a user at the beginning of each phase,
   - heart rate of said user at the end of each phase,
   - minimum heart rate reached of said user during each phase,
   - maximum heart rate reached of said user during each phase,
   - cumulated heart rate reached of said user during each phase,
   - minimum work load applied during each phase,
   - cumulated work load applied during each phase.

Generally, it is known that cardiovascular rehabilitation is an essential factor in primary and secondary prevention of cardiovascular disease. Primary prevention refers to the prevention of a disease in a normal population (supposed not having the disease). Secondary prevention refers to patients with proven cardiovascular disease, e.g., patients after recent cardiac event, after bypass surgery, after valvular replacement. Secondary prevention is the prevention of a disease worsening or preventing new events to occur. It has been clearly established that exercise and fitness reduce Coronary Heart Disease and that improving physical condition is as powerful as changing other major cardiovascular risk factors. There is a strong dose-response relationship. This is true for both sexes and as recently demonstrated for all ages, this means that exercise training is very important for the elderly, making this huge population a particular target for cardiovascular rehabilitation.

It has also been demonstrated that occupational and leisure activity has some beneficial effect in the normal population. But data from the literature also show a paradox : higher risk patients require greater level of training in order to get benefit from cardiovascular rehabilitation. However the practice shows that those patients do not apply for such rehabilitation programs.

Several methods for adapting parameters of a training device have been developed. Among them, US 4 860 763 describes a method, in which, upon instructions from the user or from a physician, control means updates exercise means and incrementally increase the work load. This method comprises the overwriting of data collected during an exercise and the adaptation of the training data directly upon the basis of the data collected during said exercise.

But this method is insufficient for having a successful cardiovascular rehabilitation. Indeed, the revalidation of cardiac patients is almost exclusively performed inside hospitals. But available resources in hospital are limited on the one hand, and on the other hand, patients who are returning to active life may not always be able or willing to conform to strict hospital schedules. Time constraint imposed by in-hospital programmes is a major factor for the low patient's compliance. Moreover, those still active patients want to remain independent from hospital. This leads to a major lack of application of cardiac, vascular and pulmonary revalidation, with serious consequences both on public health and personal prognosis.

Patients who are looking after themselves to train at home or at the gymnastic centre, sometimes with their doctor's push, will often inadequately train. This is because they either choose a training level that is too low because of apprehension, or because they set themselves at an overly high training level, which could be dangerous for their health. Also the setting of the device are often too elaborated for most patients : too complicated on the one hand and not fully adapted on the other hand.

Consequently, in such a device, a bidirectional link between the physician (usually the cardiologist) and the patient's training equipment is currently lacking.

It is an object of the invention to provide a method offering a communication possibility between the physician (usually the cardiologist) and the patient's training equipment.

To this end, the method according to the invention, is characterised in that it comprises the steps of :
- recording a session data set, comprising a session identifier and said captured data set, and said training data set into a memory provided for storing said training data set and said session data set of a performed session,
- comparing the session data set of said performed session with the training data set and verifying an effective performing of the training session.

By providing these steps, a bidirectionnal link between the specialist in cardiovascular disease, or in sport medicine or the like and the cardiovascular training device used by the patient is established. The steps as described herebefore offers a monitoring loop, between the doctor and the patient. Such steps will dramatically improve the efficiency of the training and the security for the patient. The steps achieve the best possible results when training according to the training set-up prepared by the physician. The training set-up, as described before, is stored into said memory, in particular formed by a memory card, which is inserted by the patient in the training device to ensure that the latter will be correctly programmed (since being programmed by the physician) and that the training set-up is adapted to the patient. Moreover, when the patient is training, a data set is captured during the training session forming a session data set, which is stored into the memory for being checked by the physician latter. In addition, the physician is able to verify if the training session have really been performed and what happened during the performed session in terms of heart rate and the like.

It should be understood that cardiovascular and pulmonary revalidation is based on a training mode called endurance, in opposition of an isometric mode. In the endurance mode, the goal is to maintain a target rate during a predetermined time-span. The typical training tool for cardiovascular training is the static bicycle in Europe or the treadmill in U.S.A., which is coupled to a heart rate monitor, an indispensable device which makes it possible for the patient to control his heart rate throughout the effort phase. After a complete medical evaluation, taking into account a series of parameters, the cardiologist is able to propose a tailor made training set-up to his patient. Once this evaluation has been performed, the cardiac, vascular or pulmonary patient can be provided with a very precise training program (training set-up).

Moreover, since the memory records the session data (captured data) during a performed session, the physician is able to monitor the training session in order to check if the scheduled program has been correctly followed and effectively done.

In a particular embodiment according to the invention, the method further comprises the steps of:
- analysing an evolution of at least one of said captured data set,
- updating the training data set on the basis of said evolution of said captured data set, and
- adapting the training set-up by inputting updated training data set.

Cardiac, pulmonary and vascular patients are physically limited, by their heart status and sometimes by their co morbidities. They are also psychologically hearted and fear exercise training. A strong factor for succeeding in training those patients is the care taken by the physician understanding the patient's problems and the confidence of the patient in the safety of the exercise training. This confidence can tremendously be enhanced when the physician will be able to set himself a customised program for his patient and to clearly and easily monitor the training sessions.

Thanks to the feature just mentioned before, the physician is able to adjust the subsequent sessions according to the evolution (improvement or worsening) of the medical parameters of the medical patient and the medical knowledge in the field of cardiovascular prevention by exercise and thereby installing the patient confidence.

In a particularly preferred embodiment according to the invention, the method further comprises the steps of:
- comparing the captured data set with statistical data originating from a statistical data base for cross-comparison between different patients, in particular suffering from a similar pathology, and/or taking a similar medication
- updating the training data set on the basis of said statistical data,
- adapting the training set-up by inputting updated training data set.

It should be envisaged that a normal patient heart rate profile and evolution is very different of the one of a cardiac, pulmonary or vascular patient.

Thanks to the feature just mentioned before, the physician is able to adjust the subsequent sessions according to the evolution (improvement or worsening) of the medical parameters of the medical patient and according to statistical data coming from patients suffering from a same or a similar pathology. This allows the physician to really customise the training set-up.

The invention also relates to a device for carrying out the method according to the invention. The device according to the invention comprises:
- means for composing a training cycle comprising :
   - a body warming-up phase having a warming-up phase time duration and a warming-up phase target heart rate to be determined by a physician,
   - at least one body training phase having a training phase time duration, an exercise profile and a training phase target heart rate to be determined by said physician,
   - a body recovery phase having a recovery phase time duration and a recovery phase target heart rate to be determined by said physician;
- means for determining a number of subsequent training cycles, said number of cycles forming a training session having a session time duration;
- means for determining said warming up phase target heart rate, said training phase target heart rate and said recovery phase target heart rate for a user and regulation parameters for the training device;
- means for forming a training data set by associating said warming-up phase time duration, said warming-up phase target heart rate, said training phase time duration, said profile of exercise, said training phase target heart rate, said recovery phase time duration, said recovery phase target heart rate, said number of subsequent cycle, said session time duration and said regulation parameters;
- means for supplying said training data set to said training device;
- means for converting said training data set in order to form a training set-up using said regulation parameters for regulating a work load to be applied to said training device;
- means for monitoring of a biomedical parameter, in particular a heart rate, when performing said training session;
- means for collecting a data set captured during said training session, said captured data set comprising:
   - effective duration of each phase, each phase being either the warming up phase, the at least one training phase and the recovery phase,
   - heart rate of a user at the beginning of each phase,
   - heart rate of said user at the end of each phase,
   - minimum heart rate reached of said user during each phase,
   - maximum heart rate reached of said user during each phase,
   - cumulated heart rate reached of said user during each phase,
   - minimum work load applied during each phase,
   - cumulated work load applied during each phase.
the device according to the invention is characterised in that said device further comprises:
- means for recording a session data set, comprising a session identifier and said captured data set, and said training data set into a memory provided for storing said training data set and said session data set of a performed session;
- means for comparing the session data set of said performed session with the training data set and for verifying an effective performing of the training session.

The means for recording session data and the training data is, for example, a memory, which advantageously materialises a link between a program on a doctor's computer desktop and a second program into a training device itself. This link intends to achieve the best possible results when training a patient according to the program prepared by his doctor by the storing of the training set-up designed by the physician and adequately adapted to the patient into the memory. It will be understood that the key parameter which sets the parameters of the training device is the heart rate of the patient. The user friendliness of the device is based on a plug and play mode without complex intervention of the patient or the doctor.

Another advantage of the device according to the invention, which is reached by the presence of a memory, is to monitor the training sessions in order to check if the scheduled program has been followed correctly and to adjust the subsequent sessions according to the evolution (improvement or worsening) of the medical parameters of the patient and the medical knowledge in the field of cardiovascular prevention by application of physical exercises. The key point of this part of the system is the possibility given to the doctor in charge to customise a training profile (steady state training, interval training...) and to verify the effective performing of the training session.

It will be apparent for those skilled in the art that the memory, can take the form of a personal memory card, preferably at a size of a credit card. However the physical link, being the memory, between the doctor and the training device can be replaced by an Internet link, either directly from the doctor's computer to the training device or via an intermediate web site.

The cornerstone is to always insure a bi-directional characteristic of the system: the doctor should always be able to control the parameters of the training device and the data recorded from the performed sessions by the patient should reach the doctor's computer.

This data can reach its target (doctor or training device) either by online monitoring or by offline monitoring.

In case of a direct connection from the doctor's office to the training device, various modalities can be proposed according to both sides desires:
· Online monitoring: this will not be recommended in general but might be useful for special difficult cases or to initiate a rehabilitation program for medically difficult or very anxious patients.
· Offline monitoring: the data of the performed sessions will be directly transferred into the doctor's computer for storage purpose, for example, directly in the patient's personal file, giving the opportunity for the doctor to analyse the data at his best convenience and to adjust the parameters immediately from his computer into the training device, via the internet connection (the internet module for this program can be one of the many commercially available modules performing this task).

In case of a connection via an internet web site, the data recorded from the sessions performed by the patient are stored on a control web centre site. A series of filters will help the control web centre to track the not properly performed sessions or sessions showing medical or technical problems. The manager can then contact, by either way possible, the doctor in charge to modify the program or to take any medical decision. In the case that nothing special was reported, which will be the vast majority of cases, the data of the performed sessions are immediately forwarded to the doctor in charge, directly in the patient's personal file.

It should also be envisaged that both Internet connection and the memory (card) can work together. One modality does not exclude the other. This still gives the patient the opportunity to physically bring back his memory (card) to the doctor at the next medical visit even if the data were already transferred via the Internet connection.

Other embodiments of the device or the method according to the invention are mentioned in the dependent annexed claims.

Other characteristics and advantages of the invention will appear more clearly in the light of the following description of a particular non-limiting embodiment of the invention, while referring to the drawings.

In the drawings:
Figure 1 is a schematic representation of different phases of a typical training cycle;
figure 2 is a schematic representation of a training session comprising a plurality of cycles;
figure 3 is a schematic diagram of the regulation device according to the invention; and
figure 4 is a schematic diagram of the chronological steps of a preferred embodiment of the method according to the invention.

In the drawings, a same reference sign has been allotted to a same or analogous element.

Before describing the drawings, a description of the four databases involved in the invention will be given

Those databases are located, for example, at the doctor's office in his computer device.
1. DB1: desktop database
2. DB2: patient data
3. DB3: training data
4. DB4: session data

The computer device further comprises means for composing the training cycle, means for determining a number of subsequent cycles to form a training session, means for determining the target heart rates and the regulation parameters for the training device, means for forming a training data set by associating several variables as mentioned before. The computer device can still further comprise means for comparing data, means for updating data and means for adapting the training set-up.

Before explaining in more details the drawings, the databases will be described.

The doctor's desktop database contains information which is needed for the follow-up of the patients.
■ A complete history of the sessions for one single patient: the dates at which the sessions were performed, the time spent on the bike per session, data about average and maximal heart rate, type or profile of the stress, etc.
■ A statistical database for cross-comparison between patients.
■ The database of the profiles of the sessions. The profile can be customised according the physician desires, e.g. steady state profile, interval training, and much more.

The patient data are arranged according to the following fields:
· Patient UID (unique identifier assigned to each new patient)
· Patient Name (String)
· Patient Social Number (integer)
· Patient Sex (Boolean)
· Patient Birth Date (Date)
· Patient Address (String)
· Patient Telephone (String)
· Patient e-mail (String)
· Other data can be implemented according to the country's legislation or the rules in use in each place.

The training data_related to a training session includes:
· Total training duration
· Time of the warming-up period
· Time of the recovery period
· Type or profile of the exercise, (i.e. comprising one or more training phase in a cycle)
· Number of cycles
· Target heart rates for each phase and the regulation parameters for the training device
The training parameters are:
· Training Profile UID (Unique Identifier given for each new training profile)
· Training Creation Date (Date)
· Training Profile Name (String)
· Number Of Cycles (Integer)
· Patient UID (integer)
· Warm-up Target (Integer)
· Warm-up Alarm (Integer)
· Warm-up Max Speed (Integer)
· Warm-up Min Speed (Integer)
· Warm-up Time (Integer)
· Warm-up Max Load (Integer)
· Warm-up P (Float)
· Warm-up I (Float)
· Warm-up D (Float)

The same data are used for the other phases (as explained hereinafter), i.e.:
o Training High
o Training Low
o Recovery Phase

The sessions data are captured during the training sessions. The first fields are related to the session identification. The other fields will be filled with data captured during the different phases of the training session.
The session identifier comprises
· Session Date (Date)
· Session Time (Time)
· Session UID (unique identifier)
· Training UID (Integer)
· Analyzing Date (Date)
The training session data comprises
· Time1 (integer)
· BPMbegin1 (float)
· BPMend1 (float)
· BPMmin1 (float)
· BPMmax1 (float)
· BPMsum1 (float)
· Cmin1 (float)
· Cmax1 (float)
· Csum1 (float)
(wherein BPM means Beats Per Minutes)

As already mentioned, the same data are recorded for each phase of each session. It should be mentioned that the memory card includes the following databases:
1. Database A: data related to the training data set
2. Database B: data related to the performed sessions
Here is a typical example of the card content for the training description.

The values indicated on the left hand side represent a typical parameter value for the corresponding training data.
- 21: UID_patient
- 37: UID_training
- 100: Warm_up_target_BPM
- 200: Warm_up_alarm_BPM
- 40: Warm_up_speed_MIN
- 60: Warm_up_speed_MAX
- 15: Warm_up_time
- 100: Warm_up_load_MAX
- -0.100000: Warm_up_P
- -0.200000: Warm_up_I
- 0.000000: Warm_up_D
- 150: Training_HI_target_BPM
- 200: Training_HI_alarm_BPM
- 40: Training_HI_speed_MIN
- 60: Training_HI_speed_MAX
- 10: Training_HI_time
- 200: Training_HI_load_MAX
- -0.400000: Training_HI_P
- -0.200000: Training_HI_I
- 0.000000: Training_HI_D
- 100: Training_LOW_target_BPM
- 200: Training_LOW_alarm_BPM
- 40: Training_LOW_speed_MIN
- 60: Training_LOW_speed_MAX
- 10: Training_LOW_time
- 200: Training_LOW_load_MAX
- -0.400000: Training_LOW_P
- -0.200000: Training_LOW_I
- 0.000000: Training_LOW_D
- 1: Training_cycles
- 80: Recovery_target_BPM
- 200: Recovery_alarm_BPM
- 40: Recovery_ MIN speed_
- 60: Recovery_ MAX speed_
- 15: Recovery_time
- 100: Recovery_ MAX load_
- -0.100000: Recovery_P
- -0.200000: Recovery_I
- 0.0: Recovery_D

The data of both the training sessions set-up and data related to the performed sessions will have to fit into the memory of the card. On the card, the amount of memory used should be approximately 168 bytes for the training data and 128 bytes for each session data. Typical I²C memories are presently 32 or 64 KB large. Therefore, in a 64kb memory (8kbytes), it will be possible to store 1 training description (168 bytes) and 61 session records (128 bytes each).

In the subsequent description, the following terms will have the meaning as described hereunder:
Concerning the training parameters/data
   Training profile UID: a training profile is unique and has its own UID-training. This means that the training profile can easily be duplicated for different patients. The duplication setting is optional. This option can save much time for the doctor when he designed a new session program for a new patient.
   Training Creation date: creation date of the training profile.
   Training Profile Name: training profile data Name which allows the doctor to easily identify the training profile.
   Number of cycles : number of cycles during the training phase in a training session.
   Patient UID: patient for which this particular training profile is prescribed.
   Warm-up Target: Target Heart Rate for the Warm-up phase.
   Warm-up Alarm: Heart Rate upper limit for the Warm-up phase.
   Warm-up Max Speed : Upper limit for the bike rotation speed. If the limit is crossed, an alarm message will appear on the LCD display, to invite the user to slow down.
   Warm-up Min Speed : Lower limit for the bike rotation speed. If the limit is crossed, an alarm message will appear on the LCD display, to invite the user to increase speed.
   Warm-up Time : Duration of the Warm-up phase.
   Warm-up Max Load : Upper limit of the load applied during the warm-up phase. It limits the maximum load the patient will have to face. This is mainly intended for security reasons.
   Warm-up P: Proportional parameter of the bike regulator during the Warm-up phase.
   Warm-up I : Integer parameter of the bike regulator during the Warm-up phase.
   Warm-up D: Derivative parameter of the bike regulator during the Warm-up phase.
Concerning the session identifier:
   Date Session : Date when the session is carried out by the patient
   Session Time : Hour, min and sec when the session begun (HH/MM/SS)
   Session UID : Allows unambiguous identification of each session throughout the whole database
   Training UID : UID of the training description used for the training sessions
   Analysing Date : Date when the card is read (i.e. when the data are downloaded by the physician after the patient visited to his office).
Concerning the session data (data captured upon performing the exercise)
   Time1 : Duration of the training phase in seconds
   BPMbegin1 : Heart Rate at the beginning of the phase
   BPMend1: Heart Rate at the end of the phase
   BPMmin1: Minimum Heart Rate reached during the phase
   BPMmax1 : Maximum Heart Rate reached during the phase
   BPMsum1 : Cumulated Heart Rate reached during the phase. In order to obtain the Mean Heart Rate in Beats Per Minute -BPM-, this number BPMsum1 has to be divided by Time1.
   Cmin1 : Minimum Load applied during the phase
   Cmax1 : Maximum Load applied during the phase
   Csum1 : Cumulated Load applied during the phase. In order to obtain the average Load, this number Csum1 has to be divided by Time1.

The means for supplying said training data to said training device can comprise a memory, an interface or any connection between the means for forming a training data and the training device such as an internet connection or the like or a combination thereof.

The means for converting said training data set to form a training set up using said regulation parameters is for example a PID regulation device, which can be internal to the training device or a separate one or which can be a part of the doctor's computer device.

Means for monitoring a biomedical parameters is preferably a heart rate monitor, particularly a cardio-frequency meter.

Means for collecting a captured data set is preferably an interface outputting the data measured by the training device and an interface supplying the data output by the heart rate monitor.

Means for recording is preferably the memory card as mentioned herebefore.

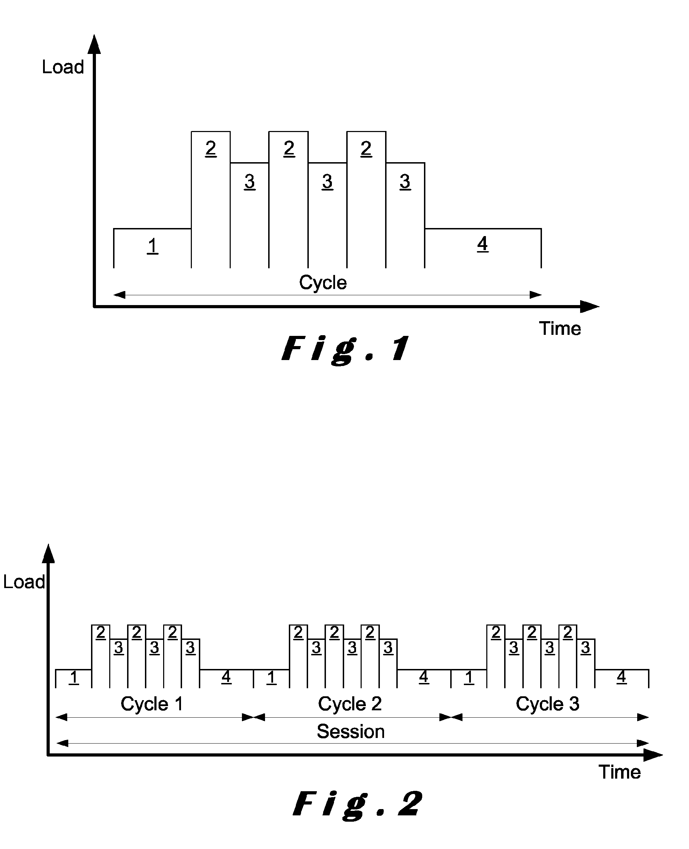

Figure 1 illustrates a composed cycle comprising a warming up period 1, having a warming-up phase time duration and a warming-up phase target heart rate, at least one body training phase 2,3 having a training phase time duration, an exercise profile 2+3 and at least a training phase target heart rate and a body recovery phase 4 having a recovery phase time duration and a recovery phase target heart rate to be determined by said physician. The training cycle as described here comprises a succession of training phases being an alternation of a high training phase 2 and a low training phase 3. It should be envisaged that the training cycle can also comprises only one training phase being a high constant or a medium constant training phase, which phase has a plateau shape.

On the graph at Fig. 1 or 2, the load has been plotted depending on the time duration. It will be understood that the load and the target heart rate are strictly and directly connected. The target heart rate (independent variable) is converted into said work load (dependent variable) either by an internal PID into the training device or by the PID regulation device according to the invention.

Figure 2 shows a training session according to the invention being a series of cycles. The cycle is as described herebefore and the session is elaborated by determining a number of subsequent training cycles.

The determination of the training cycle and the training session is the basis for the determination of the training data set, which determination comprises the additional steps of :
- determining a warming up phase target heart rate, a training phase target heart rate and a recovery phase target heart rate for a user and regulation parameters for the training device,
- forming a training data set by associating said warming-up phase time duration, said warming-up phase target heart rate, said training phase time duration, said profile of exercise, said training phase target heart rate, said recovery phase time duration, said recovery phase target heart rate, said number of subsequent cycle, said session time duration and said regulation parameters.
The training data are then transferred to said training device and converted to form a training set-up.

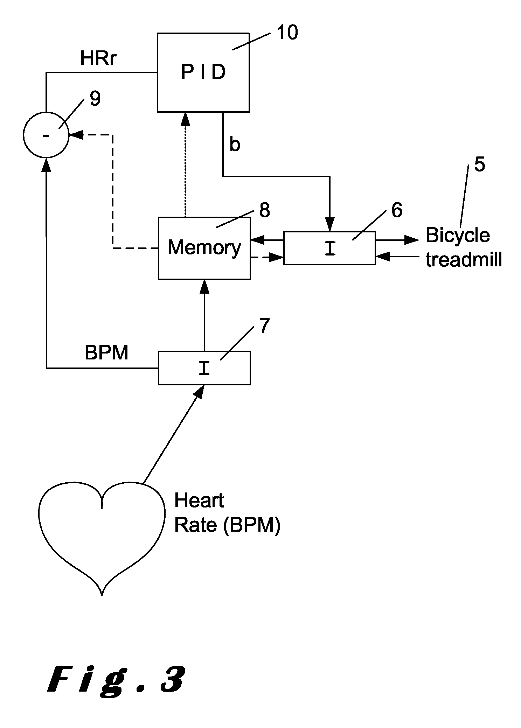

Figure 3 illustrates the regulation loop for carrying out the method according to the invention. As mentioned before, the training data set have been stored in the memory 8. The training data set stored in the memory comprises several parameters, as mentioned before, like the target heart rate for each phase, the PID parameters for each phase, the time duration for each phase, etc. The target heart rate (heavy dotted line) is output from the memory 8 to a logic member 9 for comparison with the instantaneous heart rate (BPM) of the patient. BPM is transmitted to the logic member via an interface 7 from a heart rate monitor brought by said user when performing the training session. From the comparison logic operation, performed by said member 9, a resulting signal HRr (resultant heart rate signal) is obtained, which is sent to the PID regulation device 10 in order to form a signal b.

The BPM of the user is recorded into the memory 8, via the interface 7 during the training session.

Since the regulation parameters are stored into the memory (by the physician, after being determined by either cross comparison between statistical data and patient data, by his medical knowledge or by the evolution of the session data, or the like), they are available for the PID regulation (see lightly dotted line) device 10 which outputs the signal b.

The signal b is a signal which is sent to the training device 5, through the interface 6 for adapting the exercise workload depending on the input signal (resultant HRr) and the regulation parameters (stored into the memory). The training device 5 imposes a workload corresponding to the signal b. The applied workload and the data of the effectively performed exercise are also recorded into the memory 8 as mentioned before (time, workload, speed, etc.). Some key data such as for example BPMmin1, BPMmax 1, BPMsum 1, etc. are directly determined by a logic operation in the heart rate monitor itself and sent to the memory 8. Other key data such as for example Cmax1 and Csum 1, etc. are directly determined by a logic operation in the training device itself and sent to the memory 8.

By changing the regulation parameters, it is possible to conceive a training device which responds fast or slowly in order to reach the target heart rate.

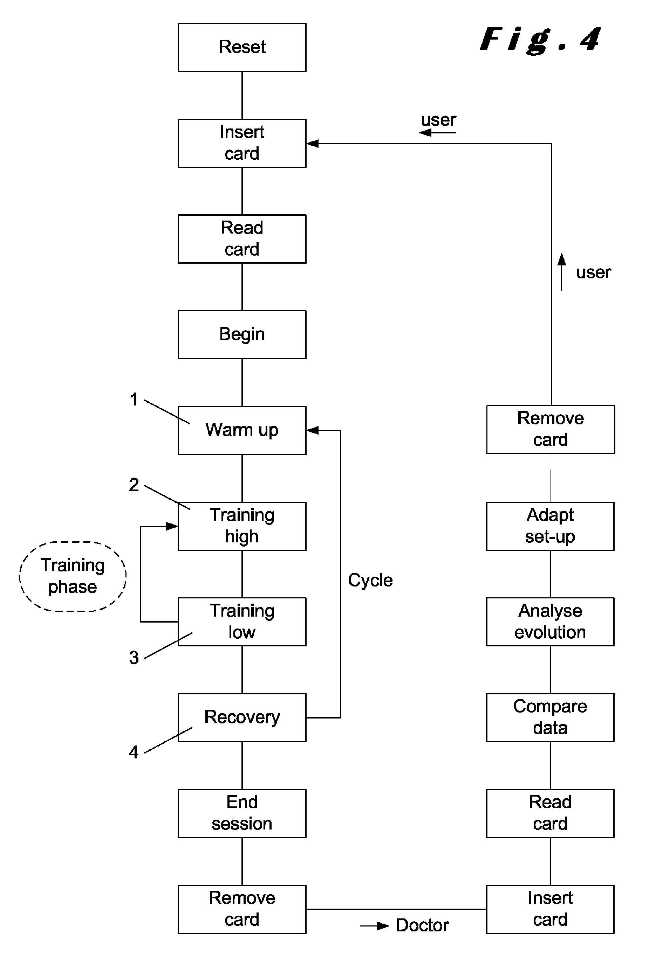

Figure 4 illustrates a flowchart of the chronological steps of the method according to the invention.

As it can be seen, the method mainly comprises:
1. The memory card is programmed by the doctor in his office and handed over to the patient.
2. Upon returning home the patient then inserts the card in his training device (e.g. bicycle or treadmill), which is then automatically programmed. Thus the patient must only perform a limited number of manipulations and, most importantly, no technical programming at all (plug and play basis). In particular, the patient does not have to introduce parameters like age, sex, and weight, level of exercise, etc, as is recommended on most available home training devices.
3. When the patient performs his training routine, several data are captured by the training device and inscribed on the card. For example: the duration of each training session, the heart rate distribution (maximal, mean, heart rate-time distribution), the applied workload (maximal, mean, heart-rate time-diagram), and, possibly, other parameters previously selected by the specialist physician.
4. On the patient's next visit to his doctor, he returns his memory card to the physician.
5. The doctor then reads the memory card via his computer using an available reading module, or via an independent module (a cradle). Then, after only a few mouse-clicks, the doctor is now able to have a complete overview of his patient's training sessions. He can now adjust what needs to be adjusted and easily reprogram the memory card for the following training period and give it back to the patient. The programmed parameters can always be adapted according to the patient's condition and the training profiles can always be adapted to the evolution of the knowledge in the field and scientific recommendations.
6. And a new cycle begins again.

Although the preferred embodiments of the invention have been disclosed for illustrative purpose, those skilled in the art will appreciate that various modifications, additions or substitutions are possible, without departing from the scope and spirit of the invention as disclosed in the accompanying claims.

## Claims

1. A method for adapting parameters of a training device comprising the steps of:
• composing a training cycle comprising :
• a body warming-up phase having a warming-up phase time duration and a warming-up phase target heart rate to be determined by a physician;
• at least one body training phase having a training phase time duration, an exercise profile and a training phase target heart rate to be determined by said physician;
• a body recovery phase having a recovery phase time duration and a recovery phase target heart rate to be determined by said physician;
• determining a number of subsequent training cycles, said number of cycles forming a training session having a session time duration;
• determining said warming up phase target heart rate, said training phase target heart rate and said recovery phase target heart rate for a user and regulation parameters for the training device;
• forming a training data set by associating said warming-up phase time duration, said warming-up phase target heart rate, said training phase time duration, said profile of exercise, said training phase target heart rate, said recovery phase time duration, said recovery phase target heart rate, said number of subsequent cycle, said session time duration and said regulation parameters;
• supplying said training data set to said training device;
• converting said training data set in order to form a training set-up using said regulation parameters for regulating a work load to be applied to said training device;
• monitoring of a biomedical parameter, in particular a heart rate, when performing said training session;
• collecting a data set captured during said training session, said captured data set comprising:
• effective duration of each phase,
each phase being either the warming up phase, the at least one training phase and the recovery phase,
• heart rate of a user at the beginning of each phase;
• heart rate of said user at the end of each phase;
• minimum heart rate reached of said user during each phase;
• maximum heart rate reached of said user during each phase;
• cumulated heart rate reached of said user during each phase;
• minimum work load applied during each phase;
• cumulated work load applied during each phase;
**characterised in that** said method further comprises
• recording a session data set, comprising a session identifier and said captured data set, and said training data set into a memory provided for storing said training data set and said session data set of a performed session; and
• comparing the session data set of said performed session with the training data set and verifying an effective performing of the training session.

2. A method according to claim 1, **characterised in that** it further comprises the steps of:
• analysing an evolution of at least one of said captured data set ;
• updating the training data set on the basis of said evolution of said captured data set; and
• adapting the training set-up by inputting updated training data set.

3. A method according to claim 1 or 2, **characterised in that** it further comprises the steps of:
• comparing the captured data set with statistical data originating from a statistical data base for cross-comparison between different patients, in particular, suffering from a similar pathology and/or taking a similar medication;
• updating the training data set on the basis of said statistical data;
• adapting the training set-up by inputting updated training data set.

4. Device for carrying out the method according to claims 1 to 3 comprising :
• means for composing a training cycle comprising :
• a body warming-up phase having a warming-up phase time duration and a warming-up phase target heart rate to be determined by a physician;
• at least one body training phase having a training phase time duration, an exercise profile and a training phase target heart rate to be determined by said physician;
• a body recovery phase having a recovery phase time duration and a recovery phase target heart rate to be determined by said physician;
• means for determining a number of subsequent training cycles, said number of cycles forming a training session having a session time duration;
• means for determining said warming up phase target heart rate, said training phase target heart rate and said recovery phase target heart rate for a user and regulation parameters for the training device;
• means for forming a training data set by associating said warming-up phase time duration, said warming-up phase target heart rate, said training phase time duration, said profile of exercise, said training phase target heart rate, said recovery phase time duration, said recovery phase target heart rate, said number of subsequent cycle, said session time duration and said regulation parameters;
• means for supplying said training data set to said training device;
• means for converting said training data set in order to form a training set-up using said regulation parameters for regulating a work load to be applied to said training device;
• means for monitoring of a biomedical parameter, in particular a heart rate, when performing said training session;
• means for collecting a data set captured during said training session; said captured data set comprising:
• effective duration of each phase;
each phase being either the warming up phase, the at least one training phase and the recovery phase,
• heart rate of a user at the beginning of each phase;
• heart rate of said user at the end of each phase;
• minimum heart rate reached of said user during each phase;
• maximum heart rate reached of said user during each phase;
• cumulated heart rate reached of said user during each phase;
• minimum work load applied during each phase;
• cumulated work load applied during each phase;
**characterised in that** said device further comprises
• means for recording a session data set, comprising a session identifier and said captured data set, and said training data set into a memory provided for storing said training data set and said session data set of a performed session; and
• means for comparing the session data set of said performed session with the training data set and for verifying an effective performing of the training session.

5. Device according to claim 4, **characterised in that** it further comprises:
• means for analysing an evolution of at least one of said captured data set;
• means for updating the training data set on the basis of said evolution of said captured data set; and
• means for adapting the training set-up by inputting updated training data set.

6. Device according to claim 4 or 5, **characterised in that** it further comprises:
• means for comparing the captured data set with statistical data originating from a statistical data base for cross-comparison between different patients, in particular, suffering from a similar pathology and/or taking a similar medication;
• means for updating the training data set on the basis of said statistical data set;
• means for adapting the training set-up by inputting updated training data set.

7. Memory card as an integral element of the device for carrying out the method according to any one of the preceding claims.
